# EUROPEAN PATENT APPLICATION

(11) **EP 4 592 267 A1**
(43) Date of publication of application: **30.07.2025**
(21) Application number: 24179125.0
(22) Date of filing: 30.05.2024
(51) Int. Cl.: C04B 35/10, C04B 35/111, C04B 35/626, C04B 35/638

(54) **COSMETIC CERAMIC MASSAGE HEAD FORMULA AND PREPARATION TECHNOLOGY THEREOF**

(30) Priority: 24.01.2024 CN 202410100139
(71) Applicant: Guangzhou Candear Packing Products Co., Ltd, Guangzhou 510000 (CN)
(72) Inventor: CAI, Guoxiang, GUANGZHOU CITY, 510000 (CN)
(74) Representative: Gale Gutierrez, Santiago

(57) **Abstract**

The invention discloses a cosmetic ceramic massage head formula and a preparation technology. The formula comprises the following raw materials: aluminum oxide fine powder, fused quartz powder, kaolin, calcium carbonate and graphene. The preparation technology comprises the steps of making ceramic slurry; injecting the ceramic slurry into a mold to make a blank, modifying and blowing the blank dry, placing the blank in a low-temperature furnace, a degreasing furnace and a high-temperature furnace separately to complete the preparation; vibrating and polishing, and soaking and cleaning to obtain a finished product. The impact strength is high, the tear resistance is good, and through adoption of the preparation technology, serious deformation with changes in temperature during preparation and explosion during high-temperature sintering are not easy to occur, so that the stability and fullness, and surface strength and abrasive resistance are improved, and accordingly the service life is prolonged.

## Description

### Reference To Prior Application

This application claims priority to Chinese Patent Application 202410100139.7, filed on January 24, 2024.

### Technical Field

The present invention relates to the technical field of cosmetic massage heads, in particular to a cosmetic ceramic massage head formula and a preparation technology thereof.

### Background

Cosmetics: preparations (other than soap) used on the human body for the purpose of beautifying, preserving or changing the appearances of persons (e.g. for performances), or used for cleansing, dyeing, scrubbing, correcting or protecting skin, hair, nails, eyes or teeth. Cosmetics are widely used by individuals for modifying or improving the appearances of persons, for example in response to fashion trends or for display of individuality. Then, the existing cosmetics are not simply applied. Sometimes the effectiveness of the cosmetics is further ensured through instruments. Therefore, massage heads emerge as the times require and mostly made of hard materials.

The prior art has the following defects or problems:
The existing cosmetic massage heads are low in impact strength and prone to tearing and deformation when being used for a long time; during the preparation process of the existing cosmetic massage heads, various problems are likely to occur as the temperature changes, such as serious deformation and explosion during high-temperature sintering, and the temperature conductivity of the product and the die-casting fullness of the product cannot be guaranteed; the product surface hardness and the abrasive resistance are relatively low.

### Summary

The present invention aims at providing a cosmetic ceramic massage head formula and a preparation technology thereof in view of the deficiencies of the prior art, so as to solve the problems raised in the background art.

In order to achieve the aforementioned purpose, the present invention provides the following technical solution: a cosmetic ceramic massage head formula comprises the following raw materials in parts by weight: 92.6% of aluminum oxide fine powder, 1.4% of fused quartz powder, 1.9% of kaolin, 2.6% of calcium carbonate, and 1.5% of graphene.

As a preferred technical solution of the present invention, the specific origin of the kaolin is Suzhou.

Another technical problem to be solved by the present invention is to provide a preparation technology of a cosmetic ceramic massage head formula, which comprises the following steps:
step one: performing feeding according to an established formula (see the ingredient list), placing the materials into a ball mill, injecting pure water into the ball mill, and stirring to make ceramic slurry;
step two: performing die-cast formation on the ceramic slurry with a metal mold, to make a blank;
step three: correcting the blank according to the drawing until it is smooth, and then blowing the blank dry;
step four: arranging bowls in layers with heat transfer crystal sand materials;
step five: feeding the bowls into a low-temperature furnace for cooling, transferring the bowls into a degreasing furnace after cooling is completed, and performing natural cooling after medium-temperature degreasing is completed;
step six: transferring the bowls into a high-temperature furnace, turning off the furnace fire after sintering, and taking the bowls out after the interior of the furnace is naturally cooled;
step seven: performing taking out after vibration and polishing are performed through stone rices;
step eight: performing soaking for 24 hours with pure water, cleaning surface residues with an ultrasonic machine, and then completing the selection to make the formula.

Further, the ball milling treatment time is 48 hours.

Further, the internal temperature of the low-temperature furnace is 300 degrees Celsius, and the low-temperature treatment time is 48 hours; the internal temperature of the degreasing furnace is 1100 degrees Celsius, and the medium-temperature degreasing treatment time is 120 hours.

Further, the internal temperature of the high-temperature furnace is 1600 degrees Celsius, and the sintering time is 48 hours.

Further, the stone rices are selected according to the shapes of workpieces and the relevant sizes of all components; the sizes are selected between 1 and 10 mm; the polishing treatment time is 72 hours.

Compared with the prior art, the present invention provides a cosmetic ceramic massage head formula and a preparation technology thereof, which has the following beneficial effects:
The cosmetic ceramic massage head formula has the advantages that a massage head composed of aluminum oxide fine powder, fused quartz powder, kaolin, calcium carbonate and graphene has high impact strength and good tear resistance, and through the adoption of the preparation technology, serious deformation with changes in temperature during the preparation process and explosion during the high-temperature sintering process are not easy to occur, so that the stability and fullness of products are effectively improved, the product surface strength and abrasive resistance are improved, and accordingly the service life of a cosmetic ceramic massage head is prolonged.

### Detailed Description Of Embodiments

The technical solutions in the embodiments of the present invention will be clearly and completely described below in conjunction with the embodiments of the present invention, and it is clear that the embodiments described are a part of the embodiments of the present invention, and not all of them. Based on the embodiments of the present invention, all other embodiments obtained by a person skilled in the art without inventive work shall fall within the scope of protection of the present invention.

In this embodiment: a cosmetic ceramic massage head formula comprises the following raw materials in parts by weight: 92.6% of aluminum oxide fine powder, 1.4% of fused quartz powder, 1.9% of kaolin, 2.6% of calcium carbonate, and 1.5% of graphene, wherein the specific origin of the kaolin is Suzhou.

Based on the cosmetic ceramic massage head formula, a preparation technology of the cosmetic ceramic massage head formula is proposed:
which comprises the following steps:
step one: performing feeding according to an established formula (see the ingredient list), placing the materials into a ball mill, injecting pure water into the ball mill, and stirring to make ceramic slurry;
step two: performing die-cast formation on the ceramic slurry with a metal mold, to make a blank;
step three: correcting the blank according to the drawing until it is smooth, and then blowing the blank dry;
step four: arranging bowls in layers with heat transfer crystal sand materials;
step five: feeding the bowls into a low-temperature furnace for cooling, transferring the bowls into a degreasing furnace after cooling is completed, and performing natural cooling after medium-temperature degreasing is completed;
step six: transferring the bowls into a high-temperature furnace, turning off the furnace fire after sintering, and taking the bowls out after the interior of the furnace is naturally cooled;
step seven: performing taking out after vibration and polishing are performed through stone rices;
step eight: performing soaking for 24 hours with pure water, cleaning surface residues with an ultrasonic machine, and then completing the selection to make the formula.

It should be noted that the ball milling treatment time is 48 hours; the internal temperature of the low-temperature furnace is 300 degrees Celsius, and the low-temperature treatment time is 48 hours; the internal temperature of the degreasing furnace is 1100 degrees Celsius, and the medium-temperature degreasing treatment time is 120 hours; the internal temperature of the high-temperature furnace is 1600 degrees Celsius, and the sintering time is 48 hours; the stone rices are selected according to the shapes of workpieces and the relevant sizes of all components; the sizes are selected between 1 and 10 mm; the polishing treatment time is 72 hours.

Finally, it should be noted that the foregoing is only a preferable embodiment of the present invention, and is not intended to limit the present invention. Although the present invention is described in details with reference to the foregoing embodiments, it is still possible for a person skilled in the art to modify the technical solutions described in the foregoing embodiments or to make equivalent substitutions for some of the technical features therein. Any modification, equivalent replacement and improvement made within the spirit and principle of the present invention shall be included within the protection scope of the present invention.

## Claims

1. A cosmetic ceramic massage head formula, **characterized by** comprising the following raw materials in parts by weight: 92.6% of aluminum oxide fine powder, 1.4% of fused quartz powder, 1.9% of kaolin, 2.6% of calcium carbonate, and 1.5% of graphene.

2. A cosmetic ceramic massage head formula according to claim 1, **characterized in that** the specific origin of the kaolin is Suzhou.

3. A preparation technology of a cosmetic ceramic massage head formula, **characterized by** comprising the following steps:
step one: performing feeding according to an established formula (see the ingredient list), placing the materials into a ball mill, injecting pure water into the ball mill, and stirring to make ceramic slurry;
step two: performing die-cast formation on the ceramic slurry with a metal mold, to make a blank;
step three: correcting the blank according to the drawing until it is smooth, and then blowing the blank dry;
step four: arranging bowls in layers with heat transfer crystal sand materials;
step five: feeding the bowls into a low-temperature furnace for cooling, transferring the bowls into a degreasing furnace after cooling is completed, and performing natural cooling after medium-temperature degreasing is completed;
step six: transferring the bowls into a high-temperature furnace, turning off the furnace fire after sintering, and taking the bowls out after the interior of the furnace is naturally cooled;
step seven: performing taking out after vibration and polishing are performed through stone rices;
step eight: performing soaking for 24 hours with pure water, cleaning surface residues with an ultrasonic machine, and then completing the selection to make the formula.

4. A preparation technology of a cosmetic ceramic massage head formula according to claim 3, **characterized in that** the ball milling treatment time in the step one is 48 hours.

5. A preparation technology of a cosmetic ceramic massage head formula according to claim 3, **characterized in that** the internal temperature of the low-temperature furnace in the step five is 300 degrees Celsius, and the low-temperature treatment time is 48 hours; the internal temperature of the degreasing furnace is 1100 degrees Celsius, and the medium-temperature degreasing treatment time is 120 hours.

6. A preparation technology of a cosmetic ceramic massage head formula according to claim 3, **characterized in that** the internal temperature of the high-temperature furnace in the step six is 1600 degrees Celsius, and the sintering time is 48 hours.

7. A preparation technology of a cosmetic ceramic massage head formula according to claim 3, **characterized in that** the stone rices in the step seven are selected according to the shapes of workpieces and the relevant sizes of all components; the sizes are selected between 1 and 10 mm; the polishing treatment time is 72 hours.
